# EUROPEAN PATENT SPECIFICATION

(11) **EP 2 231 222 B1**
(45) Date of publication and mention of the grant of the patent: **12.02.2014**
(21) Application number: 07865422.5
(22) Date of filing: 07.12.2007
(51) Int. Cl.: A61M 1/10

(54) **MEDICAL DEVICE**
MEDIZINISCHE VORRICHTUNG
DISPOSITIF MÉDICAL

(43) Date of publication of application: 29.09.2010
(73) Proprietor: NuCardia, Inc., El Dorado Hills, CA 65762 (US)
(72) Inventor: SHIFFLETTE, Michael, J., Alachua FL 32615 (US)
(74) Representative: Mergel, Volker
(86) International application number: PCT/US2007/086876
(87) International publication number: WO 2009/073037

(56) References cited:
- WO-A-03/103745
- WO-A-2006/051023
- WO-A-2007/112033
- US-B1- 6 533 716

## Description

### Field of the Invention

The present invention relates to cardiac-assist devices in general, and, more particularly, to blood pumps suitable for percutaneous insertion into the vascular system.

### Background of the Invention

Acute heart failure is the sudden inability of the heart to fill with or pump a sufficient volume of blood. The afflicted patient may become weak and short of breath and, in some instances, die. In the most severe acute heart failure episodes, the patient may suffer from cardiogenic shock, a condition that is associated with high reported mortality rates.

Acute heart failure occurs in a variety of contexts. For example, some patients who are hospitalized for Acute Coronary Syndrome (i.e., heart attack and unstable angina) develop acute heart failure. Furthermore, some open-heart surgery patients develop acute heart failure. Acute heart failure also complicates certain illnesses. Additionally, some patients who undergo a Percutaneous Coronary Intervention or other procedure are at risk for developing acute heart failure or dying.

Acute heart failure does not necessarily progress to chronic heart failure or death; recovery is possible. Many patients who have acute heart failure and those at risk for developing it receive interventions that are intended to temporarily assist the heart during a recovery period. The intervention typically lasts for less than a week, but may last longer.

These interventions may include pharmaceuticals and medical devices, including cardiac-assist devices. When these cardiac-assist devices include a pump that supplements the heart's pumping action, they often are referred to as "blood pumps." An effective cardiac assist device assumes some of the heart's pumping function, thereby unloading the heart and enabling it to recover. Cardiac-assist devices and blood pumps can be temporary or permanent.

The most common temporary cardiac-assist device is the intra-aortic balloon pump ("IABP"). An IABP is an inflatable balloon attached to a catheter. The IABP is inserted percutaneously (minimally invasively) into a peripheral vessel and advanced to the descending aorta. When the balloon inflates, it increases blood flow to the coronary arteries. When it deflates, it decreases the pressure against which the heart must pump. The IABP does not, however, increase the cardiac output significantly and a substantial percentage of cardiogenic shock patients that are implanted with IABPs die.

Other temporary cardiac-assist devices include extracorporeal (outside the body) blood pumps. Some of these devices require cardiac surgeons to connect them to the patient's heart and blood vessels directly through the exposed chest using "cannulas," which are relatively large-diameter tubes. Such procedures are considered invasive, may require cardiopulmonary bypass, and are associated with significant complications. Some other extracorporeal blood pumps are connected to the patient using relatively wide catheters or cannulas that are inserted through peripheral blood vessels. Certain of these devices do not increase the heart's output significantly, are difficult to use, and/or are associated with significant complications.

Emerging data indicate that temporary, percutaneously-inserted blood pumps may provide an alternative to IABPs as well as extracorporeal blood pumps and other cumbersome devices. When these temporary blood pumps are attached to a catheter, they are known as "catheter blood pumps." Some catheter blood pumps are inserted using established cath-lab techniques. These techniques are less invasive than cardiac surgery or other relatively complicated procedures.

Notwithstanding its attractiveness as a less-invasive alternative, certain percutaneously-inserted blood pumps exhibit one or more of the following drawbacks, in addition to any other shortcomings:
- limited pump flow;
- some degree of hemolysis (i.e., destruction of red blood cells);
- require the use of a large catheter/cannula, with a risk of ischemia; and
- relatively high cost.

The limited pump flow results from the fact that to be inserted percutaneously, the blood pump must be quite small. In particular, it is desirable for the blood pump to have a 12 French (4 millimeter) or smaller catheter.

Hemolysis can result when blood comes into contact with rapidly rotating elements.

One approach in the prior art to the size/flow challenge is the "expandable" blood pump. This pump, which is suitably small for percutaneous insertion, expands once in place within the vasculature or the heart. Although interesting conceptually, the expandable blood pump has proven to be difficult to implement. The pumps disclosed in U.S. Pat. Nos. 4,753,221, 5,749,855, and 6,981,942 are representative of the expandable blood pump and the problems of its implementation.

The pump that is disclosed in the '221 patent includes a catheter having an expandable propeller that is disposed in its distal end. When the propeller is deployed for operation, it spans a distance that is greater than the diameter of the catheter. To deploy the propeller, therefore, the distal end of the catheter must be enlarged. For that purpose, the distal end of the catheter is formed from a flexible material that is capable of expanding outward to provide a flared, enlarged-diameter region that can accommodate the propeller. The end of the catheter is enlarged by inflating a balloon that is disposed on the exterior of the catheter, just proximal to the flexible region. In particular, the tension on the distal end of the catheter resulting from the inflating balloon causes the enlargement.

Depending upon the orientation of the pump within the heart, blood is either: (1) drawn through the catheter and expelled at its distal end (near the pump), or (2) drawn in at the distal end of the catheter near the propeller, pumped through a length of the catheter, and then expelled through orifices. In either case, pumping the blood through the relatively smaller diameter catheter substantially defeats the purpose of providing an expandable pump. The region for flow must be expanded, as well as the propeller, to reap the benefits of increased flow.

The pump that is disclosed in the '855 patent has a drive cable that is surrounded, near its distal end, by an expandable cage. The distal end of the drive cable terminates in a spherical protuberance or "ball." This ball is received in a socket that is formed in the distal end of the cage. This ball and socket arrangement serves as the distal bearing of the pump. The proximal end of the cage merges into a sleeve that surrounds the drive cable. In the absence of an applied, axially-directed force, the cage remains in a collapsed state. In this state, the cage has a cylindrical form that closely surrounds the drive cable and enables the pump to be inserted into a catheter.

An outwardly foldable propeller depends from the drive cable a short distance from its distal end. In the absence of an applied, axially-directed force, the propeller remains folded flat against the drive cable. The drive cable consists of an inner part (which extends distal to the propeller and terminates in the ball, as discussed above) and an outer part (which ends at the propeller). The drive cable is designed so that its inner part is movable relative to its outer part. As the inner part of the drive cable is drawn in the proximal direction by an axially-applied force (e.g., by a medical practitioner tugging on the inner part), relative movement between the inner and outer parts of the drive cable expands the propeller. At the same time, and by virtue of the same applied force, relative movement between the sleeve and the outer part of the drive cable expands the cage. The deployed propeller can then freely spin within the expanded cage.

While this pump overcomes the aforementioned flow-restriction problem with the '221 patent, it suffers from several other significant drawbacks. One drawback pertains to its distal bearing. This bearing, which stabilizes the propeller within the expandable cage, is implemented as a thrust bearing. That is, in operation of the pump, the bearing is placed in tension as the inner part of the drive cable is moved axially (and subsequently held in place) to expand the cage and propeller. It will be appreciated that under tension, this bearing is difficult to seal. And any blood that enters the space between the ball (which is rapidly spinning since the cable is spinning) and the socket will be hemolyzed and otherwise disrupted, making this bearing a likely site for thrombosis. Furthermore, the sliding friction between the ball and socket results in heat and wear. The heat can damage the blood and the wear can generate particulates.

A second shortcoming with the blood pump that is disclosed in the '855 patent relates to the fact that the drive cable experiences an axial load (to keep the cage expanded). This axial load increases the severity of wear between the drive cable and the surrounding catheter since both will be generally non-rectilinear (and thus establish loci of high surface contact pressure) to reach the heart from the insertion point at the femoral artery.

A third drawback of this blood pump is that it requires at least three seals: one for the distal bearing, a second for the two-part drive cable, and a third for the sleeve and outer part of the drive cable. Every seal presents a possibility for leakage of blood past the seal, which can ultimately result in hemolysis and thrombosis.

A third prior-art blood pump, as disclosed in the '942 patent, expands via inflation. In particular, the pump includes an inflatable propeller and a surrounding Inflatable housing. As the housing inflates, it expands outwardly as well as inwardly. Since there appears to be nothing to restrict inward expansion of the housing, the inflated housing and the inflated propeller are likely to come into contact with one another. Contact between the rotating propeller and the housing could interfere with proper pumping function.

Additional prior-art expandable blood pumps have been disclosed in U.S. Pat. No. 6,533,716 and published patent applications WO 03/103745, US 2008/0103591, and US 2008/0114339.

The problems with prior-art expandable blood pumps, as exemplified by the shortcomings of the three devices discussed above, limit their potential utility as a life-sustaining device. As a consequence, cardiac specialists and the patients that they treat would benefit from improvements to percutaneously-inserted, expandable blood pumps.

### Summary of the Invention

The present invention provides a way to temporarily assist the heart by supplementing the heart's pumping action without some of the costs and disadvantages of the prior art.

The illustrative embodiment of the invention is a percutaneously-inserted, expandable, cardiac-assist device. While the illustrative embodiment is intended to be for temporary use, it is possible to modify it for longer-term or permanent use.

In accordance with the illustrative embodiment, the cardiac-assist device includes a pump assembly that is deployed in the ascending aorta or the heart. In some other embodiments, however, other deployment sites may suitably be used (e.g., the descending aorta, peripheral blood vessels, even right-side locations, etc.). A torque transmission line couples the pump assembly to an extracorporeal motor. The motor, via the transmission line, drives impeller blades within the pump assembly.

Since the pump assembly is percutaneously inserted, it is advantageously sized so that it can be introduced into the vascular system (e.g., Femoral artery, etc.) via a 12-French or smaller-diameter. Historically, it has been difficult to achieve average flows greater than about 2 to 2.5 liters per minute against physiologic pressures through a 12-French catheter, which has a diameter of 4 millimeters. To that end, the pump assembly expands when it reaches its intended deployment site.

In some embodiments, the pump assembly is deployed in a patient as follows:
- An introducing tube (e.g., catheter, sheath, etc.) is inserted in the vascular system of a patient and its distal end is advanced to a location just beyond the aortic arch.
- After the second end of the introducing tube is in position beyond the aortic arch, the pump assembly is inserted into the proximal (extracorporeal) end of the tube.
- The pump assembly is advanced through the introducing tube to its distal end.
- The pump assembly is deployed by exiting the distal end of the introducing tube.

It is notable that this method can suitably be used to deploy cardiac-assist devices other than those disclosed herein, assuming that they are of suitable size. In fact, the method can be used to deploy medical devices that are not necessarily cardiac-assist devices. Furthermore, this method can be used for deployment to other locations than the ascending aorta or the heart. To the extent that the pump assembly is intended for such other locations, the various operations listed above are suitably modified, as necessary. That is, if the pump assembly is to be deployed in the descending aorta, for example, it will be understood that the introducing tube need not be "advanced to a location just beyond the aortic arch."

The pump assembly generally comprises two components: an impeller (with associated support members) and a casing. The casing is an important feature because it channels flow toward and away from the impeller, among other functions.

The pump assembly of the illustrative embodiment includes several axially-located and linearly-arranged elements including: a proximal support housing, an impeller hub, and a distal support. These elements are the aforementioned impeller support members. The torque transmission line, which is embodied as a drive shaft (in the immediate vicinity of the pump assembly), passes through the proximal support housing and is rigidly fixed to the impeller housing. Collapsible impeller blades depend from the impeller hub. Through this arrangement, the rotating drive shaft drives the impeller hub which, in turn, drives the impeller blades. When deployed and in operation, the rotating impeller blades delimit a circle ("the blade circle") having a diameter that exceeds the pump assembly's collapsed diameter by a factor that is typically within the range of about 2 to about 7, and more typically within a range of 3 to 5. These ranges are provided for the purpose of illustration, not limitation. Mechanical considerations aside, the anatomical environment of the deployed pump assembly influences the maximum theoretical enlargement factor.

A plurality of spaced-apart, rib-like elements collectively defines the casing that surrounds the impeller blades, impeller hub, and a portion of the proximal and distal supports. The casing is reconfigurable between two states: an expanded (diameter) state and a contracted (diameter) state.

In the expanded state, each rib typically has a curved, non-planar, or non-rectilinear shape. The expanded state enables the casing to accommodate the deployed impeller blades. In the illustrative embodiment, the ribs adopt an arcuate shape when in the expanded state, wherein they collectively define a substantially ellipsoid-shape casing. In other embodiments, the ribs have other non-planar shapes when in the expanded state.

In the contracted state, the ribs are substantially straight or planar and virtually in contact with the axially-aligned elements (e.g., the impeller housing, etc.). In the contracted state, the casing has its minimum diameter and exhibits a cylindrical shape. For some embodiments, the contracted state enables the pump assembly to be inserted into a 12-French or smaller introduction catheter to ease deployment within the vascular system. A membrane is disposed on a portion of the casing to provide a flow-channeling function, as previously mentioned.

It is notable that, in the illustrative embodiment, the rotating impeller hub is flanked by *non-rotating* supports: the proximal support housing and the distal support. The proximal support housing receives a proximal support ring that is the terminus of the proximal end of the casing-defining ribs. The distal support receives a distal support ring that is terminus of the distal end of the ribs. In some embodiments, one of the two support rings is configured to be movable in the axial direction while the other support ring is non movable. In some embodiments, movement in the axial direction of the one movable ring enables the casing to expand and contract.

The illustrative embodiment provides a number of advantages in comparison to the prior-art blood pumps that were discussed in the Background section. These include, among any others:
A. The removal of any axial forces to the drive cable/drive shaft for opening and closing the casing.
B. A casing that enlarges in the absence of applied force.
C. A more flexible assembly that is better able to negotiate the aortic arch.
D. A distal support that, unlike the prior art, is not rotating, such that it is better able to bear the bending loads of the casing.
These advantages are discussed more fully below.

Regarding point A, the blood pump disclosed in the '855 patent applied an axial force *directly to the drive cable*/*drive shaft* to expand the surrounding cage. In the illustrative embodiment of the present invention, no axial forces are applied to the drive cable/drive shaft to expand or contract the casing. Compared to the pump disclosed in the '855 patent, the removal of this axial force in the illustrative embodiment results in:
1. Reduced stress on the drive cable, which provides one or more of the following benefits:
   a. An increase in cable life.
   b. An increases in sheath life by diminishing the cable/sheath contact forces, wear, particulates, and heat generation.
   c. Simplification of the drive cable/shaft to a single component (rather than the inner/outer coaxial arrangement disclosed in the '855 patent).
   d. Elimination of axial displacements between the drive cable, impeller hub, and bearing, which are inherently more difficult to provide bearing support for and to seal.
   e. Permits the drive cable to be terminated at the impeller hub, thereby:
      (i) enabling improved bearing and seal configurations;
      (ii) avoiding an additional rotating component in blood contact; and
      (iii) reducing the number of seals from three to two.
2. Reduced load on the distal bearing and seal, which provides one or more of the following benefits:
   a. Eliminates the thrust load, thereby:
      (i) diminishing power losses to friction; and
      (ii) simplifying bearing design.
   b. Reduces the radial and bending load on the distal bearing since in the illustrative embodiment, the distal bearing is only required to hold the casing stationary.

Regarding point B, the prior art blood pumps required some actuation step to enlarge the casing. In contrast, the illustrative embodiment incorporates a casing that is open *unless* a restraining force is applied. In comparison to the prior art, the natural bias to the enlarged state enables:
1. The use of external forces to contract the casing, such as:
   a. A radial compressive force, as provided by an integral sheath or separate catheter.
   b. An axial tensile force, such as provided by an integral sheath, wires, or cable that couples to the *casing* to contract it.
2. The external forces are only required for delivery, but not for deployment and operation. The pump assembly can therefore be operated in a state that is free of axial external forces:
   a. Only rotational forces are involved (the spinning of the impeller);
   b. In the absence of axial forces, the drive cable/drive shaft is free to "float" within the sheath, thereby minimizing contact pressure, friction, and particulate generation.

Regarding point C-the elimination of the axial forces on the central drive cable-a relatively more flexible assembly can therefore be employed. This flexibility simplifies the process of negotiating the vascular system to deploy the pump assembly within a cardiac patient. It is notable that the drive cable does not experience axial forces during insertion and deployment, operation, or withdrawal.

Regarding point D, the stationary distal support of the illustrative embodiment is better able to bear the bending loads of the casing than the rotating distal support of that is disclosed in the '855 patent. Furthermore, the distal support of the illustrative embodiment is the full diameter of the impeller hub, which enhances stability.

### Brief Description of the Drawings

FIG. 1 depicts a partial view of the human body, showing the femoral artery leading to the heart and the placement within the body of pump assembly component **118** of cardiac-assist device **110** in accordance with the illustrative embodiment of the present invention.
FIG. 2 depicts further detail of the heart and the pump assembly component of the cardiac-assist device.
FIGs. 3A-3C depict alternative placement locations for pump assembly.
FIGs. 4A-4B depict the pump assembly in respective enlarged and contracted states.
FIG. 5 depicts further detail of the pump assembly.
FIG. 6A depicts further detail of region "A" of the pump assembly shown in FIG. 5.
FIG. 6B depicts further detail of region "B" of the pump assembly shown in FIG. 5.
FIG. 6C depicts further detail of region "C" of the pump assembly shown in FIG. 5.
FIG. 6D depicts further detail of region "D" of the pump assembly shown in FIG. 5.
FIG. 7 depicts an alternative embodiment of a pump impeller for use in conjunction with the pump assembly.

### Detailed Description

The following terms are defined for use in this Specification, including the appended claims:
- **Distal** means relatively further from a first end of a torque transmission line that connects a motor to a pump assembly in cardiac-assist device **110.** The motor is located at the first (proximal) end of the torque transmission line.
- **Proximal** means relatively closer to the first end of the torque transmission line.
- **Proximate** means "near to."
- **Axial** means an axis or direction that is coincident with a centerline (of a device) and contrary to "radial."
- **Operatively coupled** means that the operation of one device affects another device. For example, if a drive cable is "operatively coupled" to an impeller, it is capable of driving the impeller (i.e., causing the impeller to rotate). Operatively coupled devices need not be directly physically coupled.
Other definitions may be provided later in this disclosure.

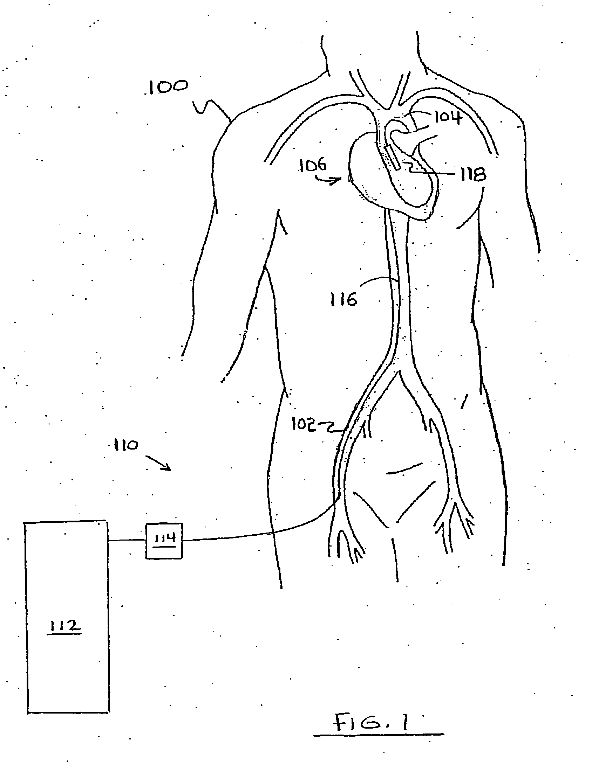

FIG. 1 depicts a silhouette of human torso **100.** Heart **106** and some internal vasculature (i.e., femoral artery **102,** aorta **104,** etc.) are shown within torso **100.** This Figure also shows portions of cardiac-assist device **110** deployed in torso **100.**

The cardiac-assist device **110** comprises controller **112**, motor **114,** torque-transmission line **116,** and pump assembly **118.** In use, some elements of cardiac-assist device **110** are extracorporeal (*i.e.,* remain outside of the patient) and others are internally deployed. More specifically, controller **112** and motor **114** remain outside of the body while the bulk of torque transmission line **116** and pump assembly **118** are deployed within the body.

Controller **112** is the human interface of cardiac-assist device **110.** The controller, which incorporates a microprocessor, typically provides one or more of the following functions, in addition to any others:
- electrical drive for the pump;
- optional fluid infusion for lubrication and for a pressurized sealing system;
- monitors system performance;
- displays impeller speed and pump flow; and
- provides auditory and visual alerts.

Motor **114** drives pump assembly **118.** In some embodiments, motor **114** is a brushless DC servomotor with speed detection and regulation. In some embodiments, motor **114** is suitable for driving pump assembly **118** to provide an average pump flow of 2.5 or more liters per minute at 60 mm Hg average pressure differential across the pump at a fluid viscosity of about 4 cP at 37°C. It will be recognized that the specific power output requirement of motor **114** will be dependent on impeller design (i.e., pump efficiency) and the diameter of the delivery system, among other factors. Those skilled in the art will know how to specify a motor as a function of system design and performance requirements.

Torque transmission line **116** operatively couples motor **114** to pump assembly **118**, thereby transmitting motor torque to the pump assembly. Torque transmission line **116** will rotate at a speed in excess of 1000 rpm and possibly as high as 50,000 rpm.

As described in further detail later in this specification, in some embodiments, torque transmission line **116** comprises a flexible drive cable and a rigid drive shaft, the former leading directly to the latter. Substantially all of torque transmission line **116** is the flexible drive cable; this flexibility is required to negotiate the vascular system. The flexible drive cable transitions to rigid drive shaft in the immediate vicinity of pump assembly **118.** The flexible and rigid portions of the torque transmission line can be "spliced" together via any of a variety of suitable known techniques, including, without limitation, swaging, via hypo tube and adhesive. In some embodiments, both the cable and the shaft are formed from stainless steel or other suitable metal.

Pump assembly **118** includes a rotating impeller that is driven by torque transmission line **116.** The rotating impeller is capable of supplementing native cardiac output by inducing flow through pump assembly **118.** As described later in this specification, the rotating impeller and surrounding casing are expandable.

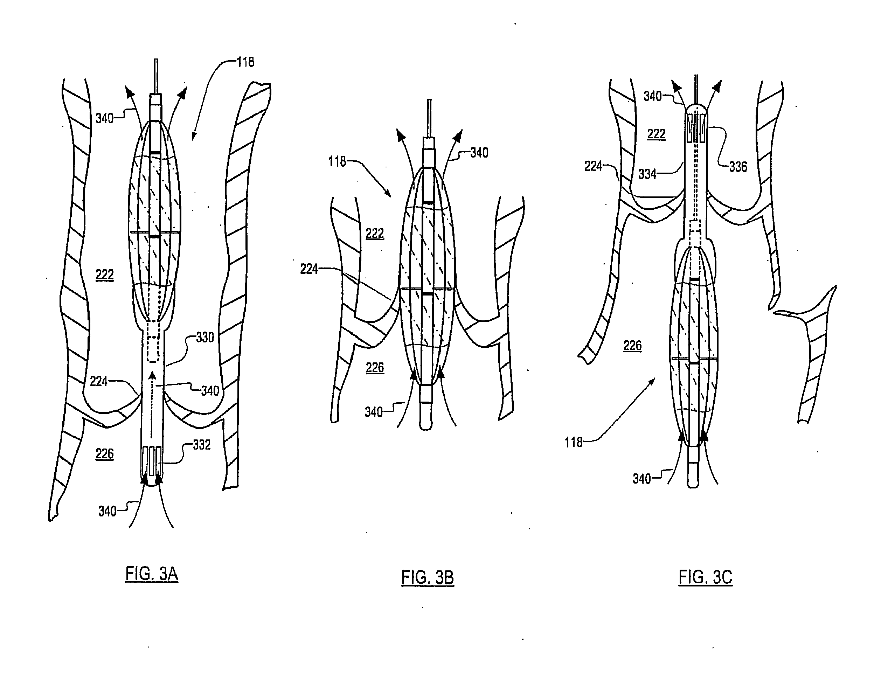

Cardiac-assist device **110** is percutaneously implanted. In some embodiments, known catheterization techniques (*e.g.,* Seldinger, *etc*.) are used to introduce pump assembly **118** into femoral artery **102,** advance it to aorta **104,** and then to its intended placement site. As discussed later in conjunction with FIGs. 3A-3C, several placement locations for pump assembly **118** are preferred. These sites include the ascending aorta, across the aortic valve, and within the left ventricle. Pump assembly **118** can be sited elsewhere, such as, without limitation, in the descending aorta, in suitably-sized peripheral blood vessels, or even in the right side of the heart or in right-side-related vasculature. At such alternate locations, one or more modifications are required to the illustrative embodiment, as described further below.

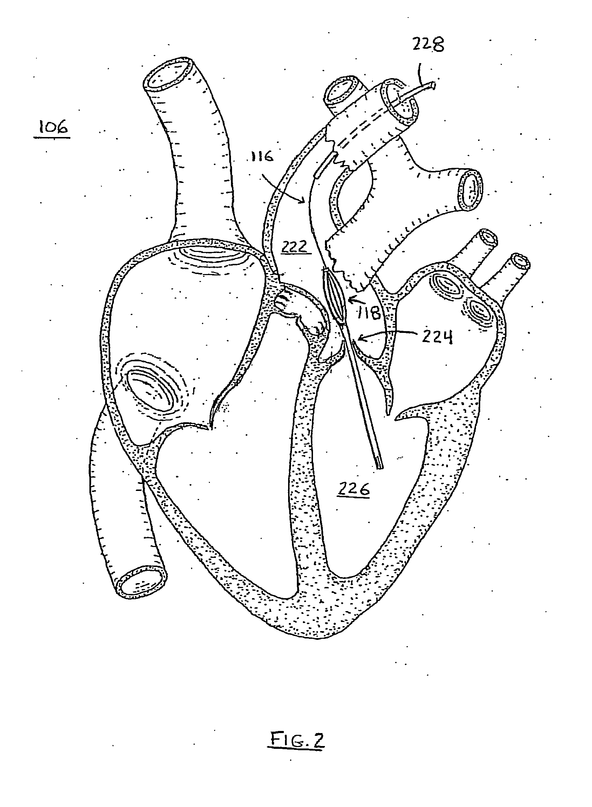

FIG. 2 depicts a view of pump assembly **118** in heart **106.** In this embodiment, pump assembly **118** is disposed in ascending aorta **222.** An extension or intake tube, which is coupled to the distal end of pump assembly **118,** crosses aortic valve **224.** In this manner, the pump assembly **118** draws its intake from left ventricle **226** and discharges blood into ascending aorta **222.** Torque transmission line **116** is shown emerging from "introducing" tube **228.** In some embodiments, the introducing tube is used to position the pump assembly at its intended position within the body.

Although the presently preferred location for pump assembly **118** is ascending aorta **118,** there are other suitable locations. FIGs. 3A-3C depict three different placement locations for pump assembly **118.**

FIG. 3A depicts further details of an embodiment in which pump assembly **118** is disposed in ascending aorta **222,** as in FIG. 2. Inflow conduit **330,** which crosses aortic valve **224** into left ventricle **226,** is advantageously coupled to the suction end of pump assembly **118.** In this arrangement, blood **340** is drawn through intake slots **332** of inflow conduit **330** due to the action of the spinning impeller within pump assembly **118.** In some other embodiments, slots **332** are not provided; rather, one or more portions of inflow conduit **330** have an open framework or are otherwise configured to permit blood to enter. Blood **340** traverses aortic valve **224** through inflow conduit **330** and then enters pump assembly **118.** Blood **340** is discharged on proximal end of pump assembly **118** into ascending aorta **222.** Note that inflow conduit **330** would typically be longer, relative to the size of pump assembly **118,** than is depicted in FIG. 3A. FIG. 2 depicts a more accurate representation of the relative lengths of pump assembly **118** and inflow conduit **330.** It is currently known to use inflow conduits in conjunction with blood pumps, so that, in conjunction with the present disclosure, those skilled in the art will know how to incorporate an inflow conduit with cardiac-assist device **110.**

FIG. 3B depicts pump assembly **118** disposed across aortic valve **224.** In the embodiment that is depicted in FIG. 3B, inflow conduit **330** is not used; blood **340** directly enters the suction end of pump assembly **118** from the left ventricle. Blood **340** crosses aortic valve **224** through pump assembly **118** and is discharged into ascending aorta **222.** In some alternative embodiments, inflow conduit **330** can optionally be used in this situation. On the one hand, it is disadvantageous, generally, to restrict the suction of a pump, such as by adding inflow conduit on its suction end. On the other hand, to the extent that inflow conduit **330** is expected to be about 10,2cm (4 inches) in length, it is less likely for the combination of the pump assembly **118** and inflow conduit **330** to be displaced from aortic valve **224** than would be the case if pump assembly **118** alone were disposed across the aortic valve.

FIG. 3C depicts pump assembly **118** disposed in left ventricle **226.** Outflow conduit **334,** which crosses aortic valve **224,** is advantageously coupled to the discharge end of pump assembly **118.** In this arrangement, blood **340** is drawn through suction end of pump assembly **118** and discharged from the pump assembly into outflow conduit **334,** which is configured identically to intake conduit **330.** Blood **340** crosses aortic valve **224** through outflow conduit **334** and is discharged into ascending aorta **222** through (optional) outflow slots **336.**

As previously mentioned, pump assembly **118** can be sited at locations other than the three that are depicted in FIGs. 3A-3C. For example, the pump assembly can be sited in the descending aorta or suitably-sized peripheral vessels. It is notable that it is preferable, but not necessary, for pump assembly **118** to take suction from the left ventricle. To the extent that the pump assembly is located in a relatively more remote location (such as the descending aorta) and is to take suction from the left ventricle, a relatively longer intake conduit will be required to reach the left ventricle (than for the embodiments that are depicted in FIGs. 3A and 3C).

Thus, in most embodiments, pump assembly **118** will be sited somewhere along the route to the left side of the heart; that is, in the descending aorta, in the aortic arch, in the ascending aorta, across the aortic valve, or anywhere within the left ventricle. It is contemplated that, with certain modifications (*e.g.,* to the impeller design, impeller rotation direction, an outflow conduit, etc.), pump assembly **118** is suitable for use in the right side of the heart or in right-side-related vasculature.

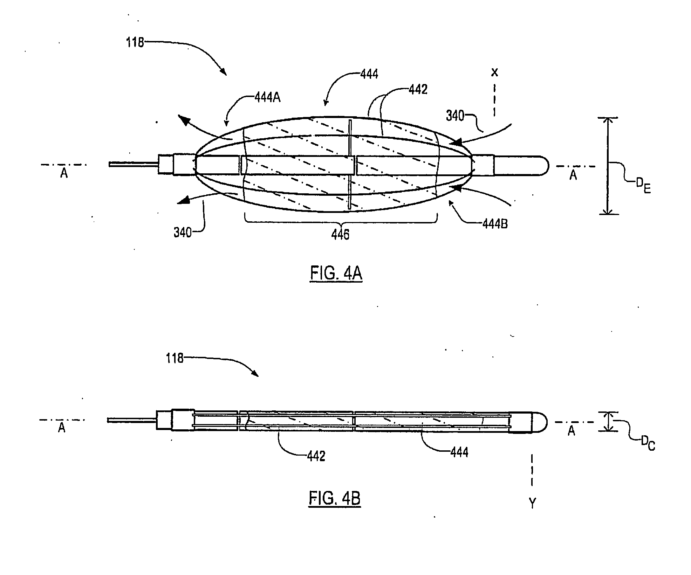

FIGs. 4A and 4B depict pump assembly **118** in respective expanded and contracted states. The expanded state is for operation (i.e., pumping blood) and the contracted state is for insertion into and withdrawal from the vascular system.

Turning now to FIG. 4A, a plurality of spaced-apart ribs **442** are axisymmetrically arranged about central axis **A-A** of pump assembly **118.** The ribs collectively define cage or casing **444.** In the embodiment that is depicted in FIG. 4A, wherein the pump assembly is in the expanded state, the ribs exhibit an arcuate shape, so that open, cage-like casing **444** adopts a typically ellipsoidal or prolate-spheroid form. In this state, the casing exhibits its maximum diameter ***D_{E}.*** This maximum or enlarged diameter is required to accommodate the impeller blades of pump assembly **118** when they are deployed for operation. As previously indicated, the ribs can exhibit any of a number of other non-planar shapes in the expanded state, as will occur to those skilled in the art in view of the present disclosure.

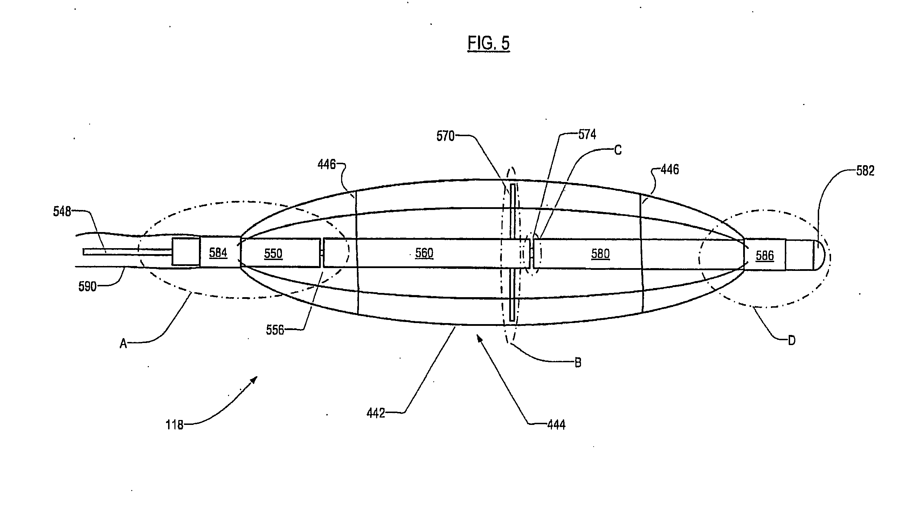

Casing **444** provides one or more of the following functions:
- it prevents the spinning impeller blades of pump assembly **118** from contacting anatomical features (*see, e.g.,* FIG. 3B);
- it establishes structural integrity (see, description accompanying FIG. 5);
- it provides a framework for an overlying membrane.

Regarding the final point above, membrane **446** covers a portion of casing **444;** end regions **444A** and **444B** of the casing remain uncovered. The purpose of membrane **446** is to channel or confine the blood in the vicinity of the impeller blades so that a flow field develops. Blood **340** enters and exits pump assembly **118** through respective uncovered regions **444B** and **444A.** In various embodiments, membrane **446** is formed from polyurethane, silicone, latex rubber, or other elastomeric compounds.

In some embodiments, ribs **442** are formed in such a way (*e.g.,* processing, materials of fabrication, *etc.*) that in the absence of a restraining force, they exhibit the aforementioned non-planar (*e.g.,* arcuate, etc.) shape, such that pump assembly **118** "naturally" assumes the expanded configuration. As a consequence, no actuating force is required to place pump assembly **118** into its operating configuration. Rather, for such embodiments, a force must be applied to *restrain* pump assembly **118** from expanding. A super-elastic material such as nitinol, etc., can be used to form ribs **442.**

FIG. 4B depicts pump assembly **118** in its contracted state. In this state, casing **444** (and pump assembly **118**) exhibits its minimum diameter ***D_{C}.*** In this state, ribs **442** are straight and substantially parallel to axis **A-A** of pump assembly **118.** Casing **444** adopts a substantially cylindrical shape.

It will be appreciated that to the extent pump assembly **118** has a relatively smaller diameter, the task of negotiating the vascular system, and in particular the aortic arch, is simplified. As a consequence, pump assembly **118** is introduced into the body (*e.g.*, the femoral artery, etc.) in the contracted state. Typically, it is after pump assembly **118** has passed the aortic arch and entered the ascending aorta or other final locations that casing **444** is expanded for operation.

In some embodiments, pump assembly **118** is deployed into the vascular system via an "introducing" tube, such as a catheter, sheath, or the like (see, *e.g.,* FIG. 2 at **228**). In some embodiments, the introducing tube is inserted into the patient *sans* pump assembly **118.** In embodiments in which pump assembly **118** is to be deployed in the ascending aorta or the heart, the introducing tube is inserted into the vascular system and advanced beyond the aortic arch. Only after the introducing tube has cleared the aortic arch is pump assembly inserted therein. Since pump assembly **118** is not present on initial introduction of the tube, it is easier for the tube to negotiate the vascular system. And, once the introducing tube is in place, it is expected to be easier for pump assembly **118** to be advanced to its intended deployment site through that tube than via other methods of delivery.

In some embodiments, the wall of the introducing tube provides the restraining force to maintain casing **444** in the contracted state. To expand casing **444,** pump assembly **118** is simply advanced beyond the distal end of the tube. To provide this functionality, the introducing tube must possess a suitably radially-inelastic wall. Standard catheters are suitably radially-inelastic for this purpose. In conjunction with the present disclosure, it is within the capabilities of those skilled in the art to provide an introducing tube having a suitably radially-inelastic wall to maintain casing **444** in the contracted state.

To collapse casing **444** for extraction from the vascular system, pump assembly **118** is drawn back into the introducing tube (or into an "extracting" tube if the introducing tube has been removed). This is implemented, in some embodiments, by fixing one end of casing **444** so that it is immovable while the other end remains free to move. For example, in FIGs. 4A and 4B, the distal end of casing **444** is movable in the axial direction along axis **A-A** and the proximal end of casing **444** is non-movable.

As shown in FIG. 4A, when pump assembly is in the expanded state, the distal support ring of casing **444** is disposed at axial position ***x*.** If pump assembly **118** were to be pulled back into the distal end of the introducing tube (i.e., if it were moved to the "left" in FIG. 4), the freely movable distal end of casing **444** would permit ribs **442** to collapse as they encounter the substantially radially-inflexible tube. As the ribs collapse, they lengthen, such that the distal end of casing **444** moves to the right along axis **A-A** to axial position ***y*** in FIG. 4B. In some other embodiments, the proximal end of casing **444** is movable along axis **A-A** and the distal end of casing **444** is non-movable.

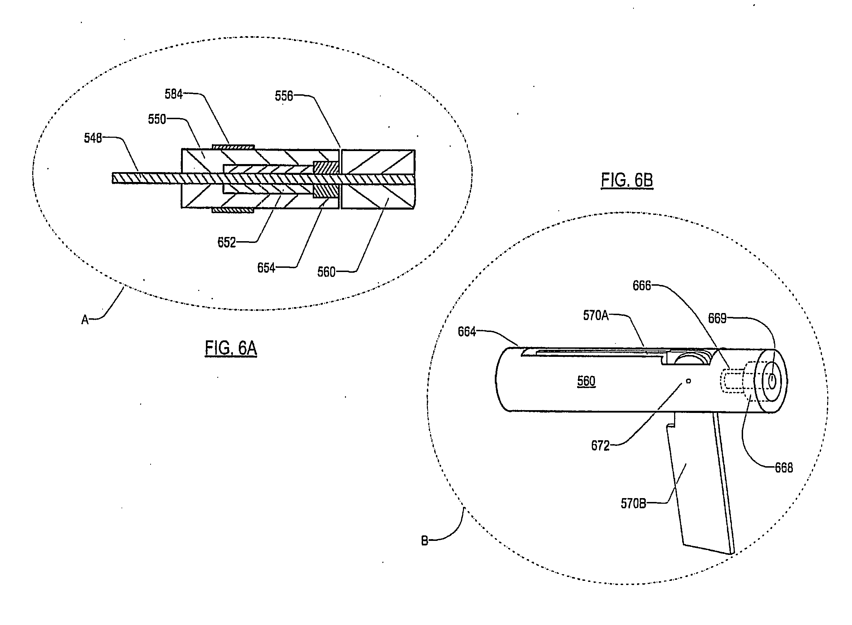

FIGs. 5 and 6A through 6D depict further details of pump assembly **118.** Referring now to FIG. 5, pump assembly **118** includes proximal support housing **550,** impeller hub **560,** impeller blades **570,** distal support **580,** nose cone **582,** plurality of ribs **442** that define casing **444,** proximal support ring **584,** distal support ring **586,** and membrane **446.**

Elements of pump assembly **118** are coaxial and, in some cases, linearly arranged with respect to one another. In the illustrative embodiment, proximal support housing **550,** impeller hub **560,** and distal support **580** are linearly arranged. Proximal support housing **550** and impeller hub **560** are coaxial with respect to drive shaft **548.** Casing **444,** which comprises ribs **442,** proximal support ring **584,** and distal support ring **586,** is coaxial with respect to proximal support housing **550,** impeller hub **560,** and distal support **580.** In some embodiments, proximal support housing **550,** impeller hub **560,** and distal support **580** comprise injection molded polymer.

To develop pumping action, torque from the external motor (see FIG. 1, motor **114**) must be delivered to impeller blades **570.** This is done by operatively coupling torque transmission line **116** to impeller blades **570.** In the illustrative embodiment, this is accomplished in the following manner.

Drive shaft **548** enters proximal end of pump assembly **118.** The drive shaft is the distal portion of torque transmission line **116.** Drive shaft extends a relatively short distance (less than about 3 centimeters) proximal of pump assembly **118.** The proximal end of drive shaft **548** transitions to a drive cable (not depicted in FIG. 5), which serves as the major portion of the torque transmission line and extends to motor **114.** The drive cable is flexible to enable it to be easily advanced beyond the aortic arch, if required. In contrast, drive shaft **548** is rigid, in order that the requisite seal and bearing (described further below in conjunction with the description of FIG. 6A; see bearing **652** and seal **654**) will function properly.

Drive shaft **548** passes through proximal support housing **550** to impeller hub **560.** Proximal support housing **550** provides a non-rotating support surface for the proximal support ring **584,** thereby supporting the proximal end of casing **444.** Since casing **444** does not rotate, it cannot couple to a rotating surface, such as impeller hub **560.**

Since proximal support housing **550** does not rotate but impeller hub **560** does, they are separated by gap **556.** And since drive shaft **548** passes through proximal support housing **550,** a bearing must be provided within the housing to accommodate the rotational movement of drive shaft **548.** A seal must also be provided within proximal support housing **550** to prevent blood from entering. If blood were to enter housing **550** in the small gap between drive shaft **548** and the bore that accepts it, the blood would be hemolyzed by the action of drive shaft **548.** Further details of this portion of pump assembly **118,** which is demarcated in FIG. 5 as region **A,** is depicted FIG. 6A.

Turning now to FIG. 6A, drive shaft **548** enters proximal support housing **550** and passes through bearing **652.** Seal **654** is disposed at the distal end of housing **550.** As previously described, bearing **652** accommodates rotation of drive shaft **548** and seal **654** prevents blood from entering proximal support housing **550.** The bore of bearing **652** provides substantially all of the structural rigidity for impeller blade **570/impeller** hub **560.** Materials suitable for bearing **652** include, without limitation, low friction polymers, such as Teflon® (polytetrafluoroethylene), Torlon® (polyamideimide), Rulon® (propriety polytetrafluoroethylene-based compounds), Vespel® (thermoplastic polyimide) sleeve bearings, biocompatible bearings and the like. In some embodiments, polyurethane or silicon lip seals or o-rings are used as seal **654.**

In some embodiments, drive shaft **548** is formed as an integral part of impeller hub **560.** In some other embodiments, impeller hub **560** is formed around drive shaft **548.** In any case, drive shaft **548** is rigidly coupled to impeller hub **560** to efficiently drive impeller blades **570.** Drive shaft **548** (and the drive cable) is formed of stainless steel or other materials having specific dimensions, hardness, surface finish, and radiused edges for damage-free seal insertion. Surface finish will be specified by the bearing or seal manufacturer to ensure compatibility with bearing **652** and seal **654.**

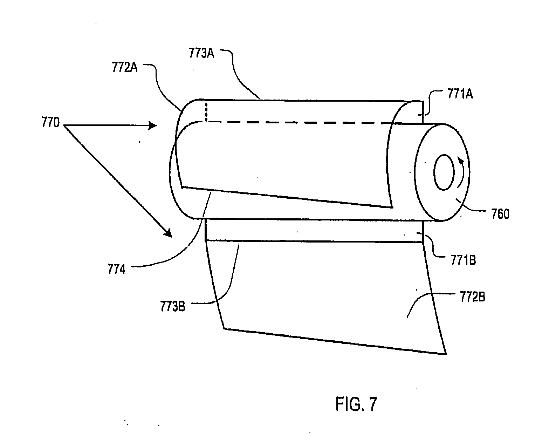

Returning to FIG. 5, impeller blades **570** are depicted in a deployed position. They extend substantially orthogonally from impeller hub **560.** Further details of this portion of pump assembly **118**, which is demarcated in FIG. 5 as region **B,** are depicted in FIG. 6B. An alternative embodiment of the impeller is described later in conjunction with FIG. 7.

FIG. 6B depicts distal end of impeller hub **560.** Slot **664** receives impeller blades **570.** Impeller blade **570A** is depicted within slot **664** in a stowed position and impeller blade **570B** is depicted in a deployed position. The impeller blades rotate about axle pin **672** to deploy or retract. Impeller blades **570A** and **570B** are formed from materials such as, without limitation, injection molded polymer or stainless steel. Axle pin **672** is formed from a material such as 300 series stainless steel. It is to be understood that the impeller blades will normally deploy and retract in unison; the stowed/deployed configuration of FIG. 6B is depicted for pedagogical purposes.

In the illustrative embodiment, axle pin **672** is near distal end of impeller hub **560** so that impeller blades **570** open from proximal to distal. In some other embodiments, axle pin **672** is substantially proximal of impeller blades **570** so that the blades open from distal to proximal.

In some embodiments, impeller blades **570** are biased to deploy; that is, they must be restrained to be kept within impeller hub **560.** In some embodiments, the biasing force partially deploys the impeller blades while the rapid rotation of impeller hub **560** completes the deployment. To the extent that impeller blades **570** are biased to at least partially deploy, ribs **442** restrain impeller blades **570** from deploying when casing **444** is in the contracted state.

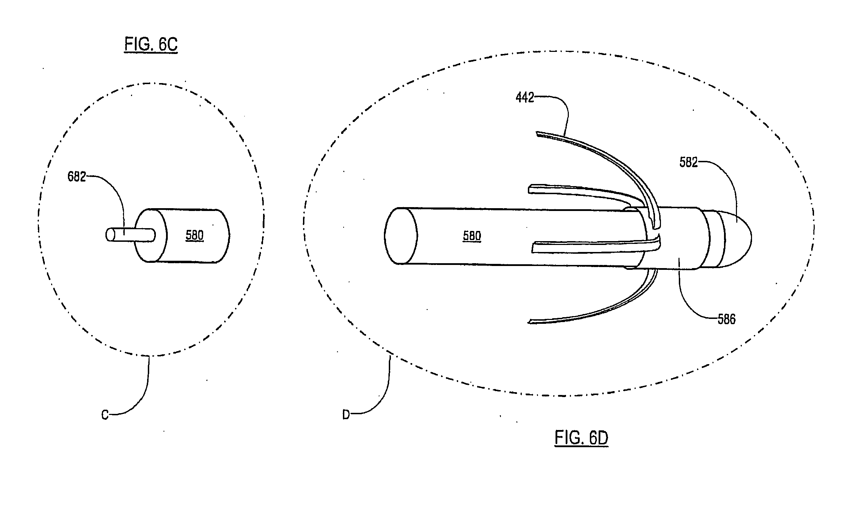

Returning again to FIG. 5, casing **444** is advantageously supported at its distal end. Such support is provided by distal support **580,** which receives distal support ring **586.** Like proximal support housing **550,** the distal support is not rotating. Since, however, impeller hub **560** is rotating, the hub and distal support **580** are separated by gap **574.** Because drive shaft **548** does not extend beyond impeller blades **570,** a pin or other means is required to couple distal support **580** to impeller hub **560.** Further details of this portion of pump assembly **118,** which is demarcated in FIG. 5 as region **C,** are depicted in FIG. 6C.

Referring now to FIGs. 6B and 6C, locating pin **682** depends from proximal end of distal support **580.** Pin **682** couples proximal end of support **580** to distal end of impeller hub **560** at bore **669.** Disposed within the distal end of impeller hub **560** are bearing **666** and seal **668.** Since, as previously disclosed, impeller hub **560** is rotating and distal support **580** is not, bearing **666** is required to accommodate this differential movement. Seal **668** prevents leakage of blood into impeller hub **560** at bore **669.** In some embodiments, bearing **666** and seal **668** are formed of the same materials as bearing **652** and seal **654,** respectively.

The distal end of distal support **580** terminates in nose cone **582,** which provides an atraumatic surface that is contoured for easy insertion and navigation through a patient's vascular system.

FIG. 6D depicts further detail of the distal end of pump assembly **118,** demarcated in FIG. 5 as region **D.**

As previously described, in some embodiments, one of either the proximal end or the distal end of casing **444** is movable in an axial direction. This facilitates the expansion and contraction of the casing. In embodiments in which casing **444** is to be collapsed simply by the act of inserting the proximal end of pump assembly **118** into an introduction/extraction catheter, then it is advantageous (but not necessary) for the distal end of casing **444** to be the movable end. In such embodiments, distal support ring **586** is coupled to distal support **580** so that it is able to readily slide along the support in either direction.

In some embodiments, such as depicted in FIG. 5, sheath **590** depends from the proximal end of proximal support ring **584.** The sheath, which runs substantially the full length of torque transmission line **116**, is used for one or more of the following purposes:
- During insertion of pump assembly **118** into a patient, tension can be placed on the sheath to maintain casing **444** in the contracted state. In such embodiments, proximal support ring **584** is movable and distal support ring **586** is fixed.
- Pressurized fluid (*e.g.,* lubricant, *etc.*) can be delivered to pump assembly **118** to provide a flow out of at least seal **654** to prevent leakage *into* that seal.

With regard to the latter point above, it is important to prevent blood from leaking into any of the seals of pump assembly **118.** To that end, in some embodiments, a pressurized fluid, such as a lubricant, is provided to seal **654** and, optionally, to seal **668.** As described above, to deliver fluid to seal **654,** the fluid can be delivered in sheath **590.** The pressurize fluid permeates the drive cable (which leads to drive shaft **548**). The fluid wets the surface of drive shaft **548** and is carried into proximal support housing **550.** The fluid passes through the small clearance between drive shaft **548** and bearing **652** and flows out through seal **654** at gap **556** (*see* FIG. 6A).

Either of several approaches can be used to provide pressurized fluid to seal **668** at the distal end of impeller hub **560.** In particular, in some embodiments, drive shaft **548** is hollow (not depicted) for conducting fluid In embodiments in which the blades collapse into the impeller hub, the hub will include impeller-blade receiving slot **664.** The fluid must be channeled around this slot, so a cross channel (not depicted) leads from the axially-disposed bore in drive shaft **548** to a second, non-axially disposed channel (not depicted) that leads to seal **668.**

In some other embodiments, rather than conducting fluid *through* impeller hub **560,** fluid is stored under pressure in a reservoir (not depicted) that is disposed in distal support **580.** In some embodiments, this fluid fills a cavity that is defined between the proximal end of distal support **580** and an internal plug (not depicted). A spring, which is positioned behind the plug within the distal support, pressurizes the fluid. For such embodiments, locating pin **682** includes a bore that communicates with the pressurized fluid reservoir. In this fashion, fluid is delivered through the bore in locating pin **682** to seal **668** in the distal end of impeller hub **560.**

FIG. 7 depicts an alternate embodiment of an expandable/collapsible impeller for use in conjunction in the illustrative embodiment of the present invention. As depicted in FIG. 7, impeller **770** includes two diametrically-opposed blade stubs **771A** and **771B** and two blades **772A** and **772B.** In the illustrative embodiment, each blade stub **771A** and **771B** extends substantially perpendicularly from and is axially aligned with impeller hub **760.** Blade **772A** is pivotably coupled to blade stub **771A** at hinge **773A.** Likewise, blade **772B** is pivotably coupled to blade stub **771B** at hinge **773B.** For convenience, these elements will be generically referenced as "blade stub(s) **771,"** "blade(s) **772,"** and "hinge(s) **773."**

Unlike impeller blades **570A** and **570B,** which retract into the impeller hub when the pump assembly is in the contracted state, impeller blades **770** simply hug the surface of impeller hub **760.** Blade **772A** is depicted in a collapsed or retracted state. To deploy for operation, impeller blades pivot about hinge **773.** Blade **772B** is depicted in the deployed or expanded state.

In some embodiments, impeller blades **772** are biased to deploy, such as by spring loading hinges **773.** In such embodiments, when pump assembly **118** emerges from an introducing tube, for example, the casing and impeller blades expand. In some other embodiments, hinges **773** are oriented so that impeller blades **772** are deployed as a consequence of the hydrodynamic Coriolis forces that arise when impeller hub **760** is rotated for operation. For the arrangement that is depicted in FIG. 7, rotation in the indicated direction will cause blades **772** to deploy. Conversely, when rotated in the opposite direction, the hydrodynamic forces push blades **772** toward the collapsed position. Hinge details are not provided in FIG. 7; it is within the capabilities of those skilled in the art, in light of the present disclosure, to design and implement hinges to provide the desired functionality.

When impeller blades **772** are collapsed, the minimum diameter of the pump assembly, excluding the casing (hereinafter "package diameter"), is the distance between hinges **773A** and **773B.** The width of each blade **772** can equal the package diameter, so that when blades **772** are deployed, their total span (*i.e.*, the diameter of the blade circle) can reach three times the package diameter.

In the illustrative embodiment, blades **772** are shown with twist. That is, edge **774** is skewed. A blade having twist can push blood axially. In some other embodiments, blades **772** do not have twist. Blades without twist will tend to push the blood radially outward in the manner of a centrifugal pump. Blades without twist are advantageously located near an opening through casing **444** (*see, e.g.*, FIG. 4A, region **444A**) so that the radially-flowing blood flows out of pump assembly **118.** In some embodiments, holes (not shown) are provided through membrane **446** to facilitate the passage of radially-flowing blood out of pump assembly **118.**

It is to be understood that the above-described embodiments are merely illustrative of the present invention and that many variations of the above-described embodiments can be devised by those skilled in the art without departing from the scope of the invention. For example, in this Specification, numerous specific details are provided in order to provide a thorough description and understanding of the illustrative embodiments of the present invention. Those skilled in the art will recognize, however, that the invention can be practiced without one or more of those details, or with other methods, materials, components, *etc*.

Furthermore, in some instances, well-known structures, materials, or operations are not shown or described in detail to avoid obscuring aspects of the illustrative embodiments. It is understood that the various embodiments shown in the Figures are illustrative, and are not necessarily drawn to scale. Reference throughout the specification to "one embodiment" or "an embodiment" or "some embodiments" means that a particular feature, structure, material, or characteristic described in connection with the embodiment(s) is included in at least one embodiment of the present invention, but not necessarily all embodiments. Consequently, the appearances of the phrase "in one embodiment," "in an embodiment," or "in some embodiments" in various places throughout the Specification are not necessarily all referring to the same embodiment. Furthermore, the particular features, structures, materials, or characteristics can be combined in one or more embodiments as defined in the claims.

## Claims

1. A cardiac-assist device having a rotatable impeller hub (560), wherein the impeller hub is operatively coupled to a torque transmission line (116) for rotation about a rotational axis and a casing (444) for enclosing an impeller blade (570) that depends from and rotates with the impeller hub, wherein the device comprises:
a non-rotating, proximal support housing (550) and a non-rotating, distal support (580) that are axially aligned with and flank the impeller hub, and wherein the distal support is coupled to the impeller hub;
a proximal support ring (584) is disposed at the proximal end of the casing and is received by the proximal support housing; **characterized in that** :
a distal support ring (586) is disposed at the distal end of the casing and is received by the distal support, and wherein one of the distal support ring or the proximal support ring is coupled to its respective support so that it is able to slide along the support in either direction;
and wherein movement in the axial direction of the one movable ring enables the casing to expand and contract.

2. The cardiac-assist device of claim 1 further comprising an extracorporeal motor (114) that is operatively coupled to the torque transmission line (116).

3. The cardiac-assist device of claim 1 wherein the casing (444) expands to a substantially ellipsoidal shape.

4. The cardiac-assist device of claim 1 wherein a locating pin couples the proximal end of the distal support (580) to the distal end of the impeller hub (550).

5. The cardiac-assist device of claim 1 wherein the proximal support housing (550) and impeller hub (560) are coaxial with respect to a drive shaft (548), wherein the drive shaft is the distal portion of the torque transmission line (116).

6. The cardiac-assist device of claim 1 wherein the casing (444) is coaxial with respect to the proximal support housing (550), the impeller hub (560), and distal support (580).

7. The cardiac-assist device of claim 1 and further wherein the proximal support housing includes a first bearing (652) and a first seal (654), wherein the first bearing accommodates rotational movement of drive shaft (548), wherein the drive shaft is the distal portion of the torque transmission line (116), and wherein the first seal (654) prevents blood from entering a region between the drive shaft and a bore that accepts the drive shaft.

8. The cardiac-assist device of claim 1 wherein the cardiac-assist device further comprises a membrane (446) that overlies at least a portion of the casing (444) and leaves end regions of the casing uncovered.

9. The cardiac-assist device of claim 7 and further wherein a distal end of the impeller hub includes second bearing (666) and a second seal (668), wherein the second bearing is required to accommodate differential movement between the rotating impeller hub (560) and the non-rotating distal support (580), and wherein the second seal prevents leakage of blood into the impeller hub, and further wherein the first seal and the second seal are the only seals required to accommodate the motion of drive shaft and impeller hub.

10. The cardiac-assist device of claim 1 wherein the torque transmission line (116) terminates in the impeller hub (560).

## Patentansprüche

1. Herzunterstützungsvorrichtung, die eine drehbare Laufradnabe (560) sowie einen Mantel (444) zum Umschließen einer Laufradschaufel (570) aufweist, wobei die Laufradnabe zur Rotation um eine Rotationsachse funktional mit einer Drehmomentübertragungsleitung (116) gekoppelt ist, und wobei die Laufradschaufel, die sich von der Laufradnabe aus erstreckt und zusammen mit dieser rotiert, wobei die Vorrichtung umfasst:
ein nicht rotierendes, proximales Halterungsgehäuse (550) und eine nicht rotierende, distale Halterung (580), die axial mit der Laufradnabe ausgerichtet sind und diese flankieren, und wobei die distale Halterung mit der Laufradnabe gekoppelt ist;
einen proximalen Haltering (584), der an dem proximalen Ende des Mantels angeordnet ist und von dem proximalen Halterungsgehäuse aufgenommen ist;
**dadurch gekennzeichnet, dass**:
ein distaler Haltering (586) an dem distalen Ende des Mantels angeordnet ist und von der distalen Halterung aufgenommen ist, und wobei entweder der distale Haltering oder der proximale Haltering derart mit seiner jeweiligen Halterung gekoppelt ist, dass er in beiden Richtungen entlang der Halterung verschoben werden kann;
und wobei durch eine Bewegung des einen beweglichen Ringes in der axialen Richtung der Mantel ausgedehnt und zusammengezogen werden kann.

2. Herzunterstützungsvorrichtung nach Anspruch 1, welche ferner einen extrakorporalen Motor (114) umfasst, der mit der Drehmomentübertragungsleitung (116) funktional gekoppelt ist.

3. Herzunterstützungsvorrichtung nach Anspruch 1, wobei sich der Mantel (444) zu einer im Wesentlichen ellipsoiden Gestalt ausdehnt.

4. Herzunterstützungsvorrichtung nach Anspruch 1, wobei das proximale Ende der distalen Halterung (580) durch einen Positionierungsstift mit dem distalen Ende der Laufradnabe (560) gekoppelt ist.

5. Herzunterstützungsvorrichtung nach Anspruch 1, wobei das proximale Halterungsgehäuse (550) und die Laufradnabe (560) koaxial in Bezug auf eine Antriebswelle (548) angeordnet sind, wobei die Antriebswelle den distalen Abschnitt der Drehmomentübertragungsleitung (116) darstellt.

6. Herzunterstützungsvorrichtung nach Anspruch 1, wobei der Mantel (444) koaxial in Bezug auf das proximale Halterungsgehäuse (550), die Laufradnabe (560) und die distale Halterung (580) angeordnet ist.

7. Herzunterstützungsvorrichtung nach Anspruch 1, wobei ferner das proximale Halterungsgehäuse ein erstes Lager (652) und eine erste Dichtung (654) aufweist, wobei das erste Lager die Antriebswelle (548) zur Rotationsbewegung aufnimmt, wobei die Antriebswelle den distalen Abschnitt der Drehmomentübertragungsleitung (116) darstellt, und wobei die erste Dichtung (654) verhindert, dass Blut in einen Bereich zwischen der Antriebswelle und einem die Antriebswelle aufnehmenden Loch eindringt.

8. Herzunterstützungsvorrichtung nach Anspruch 1, wobei die Herzunterstützungsvorrichtung ferner eine Membran (446) umfasst, die zumindest einen Teil des Mantels (444) überlagert und Endbereiche des Mantels unbedeckt lässt.

9. Herzunterstützungsvorrichtung nach Anspruch 7, wobei ferner ein distales Ende der Laufradnabe ein zweites Lager (666) und eine zweite Dichtung (668) aufweist, wobei das zweite Lager erforderlich ist, um eine Relativbewegung zwischen der rotierenden Laufradnabe (560) und der nicht rotierenden, distalen Halterung (580) aufzunehmen, und wobei die zweite Dichtung ein Eindringen von Blut in die Laufradnabe verhindert, und wobei ferner die erste Dichtung und die zweite Dichtung die einzigen Dichtungen sind, die zur Aufnahme der Bewegung der Antriebswelle und der Laufradnabe erforderlich sind.

10. Herzunterstützungsvorrichtung nach Anspruch 1, wobei die Drehmomentübertragungsleitung (116) in der Laufradnabe (560) endet.

## Revendications

1. Dispositif d'assistance cardiaque comportant un moyeu de roue rotatif (560), dans lequel le moyeu de roue est accouplé fonctionnellement à une ligne de transmission de couple (116) pour tourner autour d'un axe de rotation et une enveloppe (444) pour renfermer une aube de roue (570) qui dépend de et tourne avec le moyeu de roue, dans lequel le dispositif comprend :
un boîtier de support proximal non rotatif (550) et un support distal non rotatif (580) qui sont alignés axialement avec et qui flanquent le moyeu de roue, et dans lequel le support distal est accouplé au moyeu de roue ;
un anneau de support proximal (584) est placé à l'extrémité proximale de l'enveloppe et est reçu dans le boîtier de support proximal ;
**caractérisé en ce que** :
un anneau de support distal (586) est placé à l'extrémité distale de l'enveloppe et est reçu dans le support distal, et dans lequel un élément parmi l'anneau de support distal et l'anneau de support proximal est accouplé à son support respectif de telle manière qu'il est apte à glisser le long du support dans l'une ou l'autre direction ;
et dans lequel le mouvement dans la direction axiale dudit un anneau mobile permet à l'enveloppe de se dilater et de contracter.

2. Dispositif d'assistance cardiaque selon la revendication 1, comprenant en outre un moteur extracorporel (114) qui est accouplé fonctionnellement à la ligne de transmission de couple (116).

3. Dispositif d'assistance cardiaque selon la revendication 1, dans lequel l'enveloppe (444) se dilate pour prendre une forme substantiellement ellipsoïdale.

4. Dispositif d'assistance cardiaque selon la revendication 1, dans lequel une goupille de position accouple l'extrémité proximale du support distal (580) avec l'extrémité distale du moyeu de roue (550).

5. Dispositif d'assistance cardiaque selon la revendication 1, dans lequel le boîtier de support proximal (550) et le moyeu de roue (560) sont coaxiaux par rapport à un arbre d'entraînement (548), dans lequel l'arbre d'entraînement est la partie distale de la ligne de transmission de couple (116).

6. Dispositif d'assistance cardiaque selon la revendication 1, dans lequel l'enveloppe (444) est coaxiale par rapport au boîtier de support proximal (550), au moyeu de roue (560) et au support distal (580).

7. Dispositif d'assistance cardiaque selon la revendication 1 et en outre dans lequel le boîtier de support proximal comprend un premier roulement (652) et un premier joint (654), dans lequel le premier roulement facilite le mouvement de rotation de l'arbre d'entraînement (548), dans lequel l'arbre d'entraînement est la partie distale de la ligne de transmission de couple (116), et dans lequel le premier joint (654) empêche le sang d'entrer dans une région située entre l'arbre d'entraînement et un trou dans lequel est inséré l'arbre d'entraînement.

8. Dispositif d'assistance cardiaque selon la revendication 1, dans lequel le dispositif d'assistance cardiaque comprend en outre une membrane (446) qui recouvre au moins une partie de l'enveloppe (444) et qui laisse découvertes des régions d'extrémités de l'enveloppe.

9. Dispositif d'assistance cardiaque selon la revendication 7 et en outre dans lequel une extrémité distale du moyeu de roue comprend un deuxième roulement (666) et un deuxième joint (668), dans lequel le deuxième roulement est nécessaire pour compenser le mouvement différentiel entre le moyeu de roue tournant (560) et le support distal non rotatif (580), et dans lequel le deuxième joint empêche une fuite de sang dans le moyeu de roue, et en outre dans lequel le premier joint et le deuxième joint sont les seuls joints nécessaires pour faciliter le mouvement de l'arbre d'entraînement et du moyeu de roue.

10. Dispositif d'assistance cardiaque selon la revendication 1, dans lequel la ligne de transmission de couple (116) se termine dans le moyeu de roue (560).
